Europäisches Patentamt

European Patent Office

Office européen des brevets

(19)

(11) Publication number: **0 202 752 B1**

# EUROPEAN PATENT SPECIFICATION

(12)

(45) Date of publication of patent specification: **18.09.91**

(51) Int. Cl.⁵: **C07C 2/66**, C07C 2/86, C07C 6/12, C07C 15/24, C07C 15/14

(21) Application number: 86302656.3

(22) Date of filing: **10.04.86**

(54) Alkylation process.

(30) Priority: 22.04.85 GB 8510196
18.11.85 GB 8528382

(43) Date of publication of application:
26.11.86 Bulletin 86/48

(45) Publication of the grant of the patent:
18.09.91 Bulletin 91/38

(84) Designated Contracting States:
BE CH DE FR GB IT LI NL

(56) References cited:
EP-A- 0 009 894
EP-A- 0 094 693
FR-A- 2 163 552
US-A- 3 716 596
US-A- 4 112 056

Journal of Organic Chemistry, vol.29, 2939-2946 (1964)

Angew. Chem. Int. Ed. Engl. 27, 226-227 (1988)

(73) Proprietor: IMPERIAL CHEMICAL INDUSTRIES PLC
Imperial Chemical House, Millbank
London SW1P 3JF(GB)

(72) Inventor: Sampson, Roy John
5 Sandwood Park
Guisborough Cleveland(GB)
Inventor: Hanson, Charles Barrie
7 Primrose Close
Guisborough Cleveland(GB)
Inventor: Candlin, John Paton
4 Rudby Lea Hutton Rudby
Yarm Cleveland(GB)

(74) Representative: Bryant, Tracey et al
Imperial Chemical Industries plc P.O. Box 6
Bessemer Road
GB-Welwyn Garden City, Herts. AL7 1HD(GB)

**Description**

The present invention relates to the alkylation of multi-ring aromatic compounds, of both the condensed ring type and the carbon-linked ring type, or their derivatives.

Multi-ring aromatic hydrocarbon compounds are well-known and their properties and reactions are described in some detail in the technical literature. The alkylation of these compounds has attracted some attention in the past and a number of proposals have been made for the catalysed production of their alkylated derivatives. For example, the alkylation of naphthalene with propylene over a solid phosphoric acid catalyst has been described as well as the use of aluminium chloride promoted by amyl chloride as catalyst in the production of amyl naphthalene using pentenes as the alkylating agent. The dialkylated derivatives are valuable products and, for example, $\beta$-substituted naphthalenes and para-substituted biphenyls are useful as intermediates for monomers of high value polymers.

A number of crystalline aluminosilicates known as zeolites have been described and these materials are now finding favour as catalysts in certain reactions. For example, it has been proposed in German DE-A-3,334,084 to use a zeolite of the Pentasil-type as catalyst in a gas-phase process for the alkylation of naphthalene or alkylnaphthalenes with methanol or demethylether. However, we have found that methylation of methylnaphthalene using an H-ZSM-5 zeolite catalyst gives low yields of the desired dimethylnaphthalenes, much of the methanol being converted to olefinic, paraffinic and aromatic hydrocarbons with production also of substantial amounts of naphthalene compounds containing $C_2$ and higher substituents.

EP-B-87720 describes the isomerisation, disproportionation and transalkylation of naphthalene and methylnaphthalenes over a zeolite catalyst derived from H-ZSM-5. However, examination of the examples in this patent shows no clear $\beta$-methylation selectivity ie selectivity to the more highly desired $\beta$-substituted naphthalenes.

EP-A-94693 discloses zeolites which may be used in, inter alia, transalkylation processes and exemplifies the transalkylation of naphthalene with 1,5-dimethylnaphthalene under zeolite catalysis. However, in the exemplified process, the products are obtained in a ratio expected under conditions of thermodynamic equilibrium, no selectivity towards the $\beta$-substituted product being observed.

US-A-4112056, US-A-3716596 AND FR-A-2163552 also disclose the alkylation of aromatic hydrocarbons under zeolite catalysis. However, these documents do not disclose any selectivity in the alkylation processes disclosed therein.

Thus alkylation and transalkylation processes of the kind described in the prior art appear to have inherent disadvantages in that the alkylation or transalkylation is not very selective and a number of unwanted, low value side-products are obtained. We have now found that it is possible to improve the yield and/or selectivity to more highly desired products in processes of this kind.

According to the present invention a process for the selective production of $\beta$-substituted or $\beta,\beta$-disubstituted alkylated derivatives of naphthalene or a naphthalene derivative or 4-substituted or 4,4'-disubstituted alkylated derivatives of biphenyl or a biphenyl derivative which comprises contacting naphthalene or a naphthalene derivative or biphenyl or a biphenyl derivative with an alkylated benzene in the presence of a catalyst comprising a crystalline medium-pore zeolite or zeolitic material.

The preferred alkylated benzenes include trialkyl benzenes for example trimethylbenzenes, higher alkyl benzenes, for example ethyl-benzene and diethylbenzene, and more preferably a xylene or a mixture of xylenes. It is preferred to operate the process in the absence of alcohols and the corresponding ethers, for example methanol and dimethylether. The Applicants' experiments indicate that mixed xylenes are likely to be a preferred alkylating agent for the alkylation of naphthalene hydrocarbons.

The catalyst comprises a medium-pore zeolite or zeolitic material and by "medium pore" we mean zeolites having pores of a diameter which is at least 5Å. Examples of these preferred zeolites include zeolites of the ZSM-5 type, ZSM-11 and zeolite EU-1. Zeolite ZSM-5 has been described extensively in the technical literature, for example in US-A-3702886. Zeolite ZSM-11 has been described in US-A-3709979 and zeolite EU-1 in EP-B-42,226. In the production of $\beta$-methylated and $\beta,\beta'$-dimethylated naphthalenes and other product molecules of about this size according to the process of the invention, selectivity is best achieved by using zeolites with medium pore diameters.

The zeolite is preferably at least in part in the protonic form. Such forms of the zeolite may be prepared from forms containing metallic cations by known techniques, notably exchange with ammonium ions followed by calcination or exchange with acids, for example aqueous hydrochloric acid. In some cases, as well as protons, the cations present may beneficially include multivalent cations, for example lanthanum $(La^{2+})$ and calcium $(Ca^{2+})$.

Regeneration of the zeolite may be carried out by heating it in the presence of an oxidising gas, for example an air nitrogen mixture.

It is sometimes found that beneficial performance, for example longer catalyst life and/or higher catalyst activity, ensues when hydrogen at moderate pressures, for example in the range 2 to 20 bar, is included among the reactants. The advantageous use of hydrogen in this way is often enhanced if a hydrogenating metal, for example nickel or palladium, is incorporated into the zeolite. Where a flow system is used to operate the process of the invention, the hydrogen may be removed from the products and recycled.

The zeolite catalyst may be used in the form of suitable aggregates which are prepared by well-known techniques, for example tabletting with or without a binder, for example alumina, or by inclusion in a matrix, for example silica-alumina, in a manner analogous to that often used in the manufacture of fluid catalytic cracking catalysts.

If desired, the external surface of the zeolite may be poisoned so as to reduce or remove catalytic activity thereon, especially the catalysis of unwanted reactions, for example isomerisation of the selectively alkylated multi-ring aromatic hydrocarbons of the process. In some cases zeolites treated with magnesium and/or phosphorus compounds have beneficial properties in the process of this invention.

The process of this invention may be carried out in either the liquid phase or the gas phase either in continuous or batch operation using, for example, a fixed-bed, fluidised bed or slurry reactor. If desired, the process may also be carried out in mixed phase (liquid-gas) system and the Applicants believe that it may be advantageous to use a counter-current flow of the multi-ring aromatic reactant and the alkylating agent where one of these reactants is substantially in the liquid phase and a significant proportion of the other, prior to the reaction, is in the gas phase under the reaction conditions. The preferred reaction temperature is likely to be in the range 200 to 600° C, more preferably 300 to 500° C.

The desired product of the process may be separated from the reaction mixture in a conventional manner, for example by distillation. Unreacted reactants or undesired products, for example mono-alkylated products where di-alkylated products are desired, or vice versa, may be recycled.

In a particularly preferred embodiment of the present invention the Applicants have surprisingly found that zeolite ZSM-5 is a useful catalyst for the alkylation of naphthalene and of 2-methylnaphthalene using either xylenes or 1, 2, 4-trimethylbenzene as the alkylating agent. It is known that ZSM-5 can catalyse the methylation of naphthalene and it is presumed that the pores of ZSM-5 can accommodate the relatively small transition state involved in the direct methylation of naphthalene with methanol. However it is very surprising that the bulky transition state expected to be involved in transmethylation between xylene and napththalene or 2-methylnaphthalene can be accommodated. Indeed it is stated in the chemical literature that the pores of ZSM-5 cannot accommodate effective methyl transfer between molecules more bulky than toluene (see for example Discussions of the Faraday Soc 72 331-343 (1981)).

The process of this invention has several advantages over direct alkylation using alcohols. Thus, in methylation using methanol as the alkylating agent, a very large proportion (>80%) of the methanol is converted to a complex and non- recyclable mixture of olefins, paraffins and aromatics. On the other hand, in alkylation using xylenes the only consumption of xylene other than in the required reaction is in a small amount of disproportionation to produce 1,2,4-trimethylbenzene and toluene. The latter compounds can be recovered as a useful product or re-cycled to the alkylation process. Again, in methylation using methanol a significant amount of naphthalene or dimethylnaphthalene is ethylated or propylated by reaction with the ethylene or propylene produced from the methanol. Indeed it can happen that more can be produced of the unwanted ethyl product, for example ethyl methylnaphthalene, when the desired product is 2,6- dimethylnaphthalene. In contrast, in alkylation by the process of the invention the by-products (in this case 1-methylnaphthalene and dimethylnaphthalenes other than the $\beta$- or $\beta,\beta'$-isomers) can be recycled.

Finally, a cleaner, more easily purified product is obtained in the process of the invention than in direct methylation using methanol. The absence of ethyl- and propyl-aromatics from the methylation product of this process makes the isolation of pure, selectively methylated aromatics by distillation and/or crystallisation techniques more simple.

Thus, the alkylation of naphthalene by the process of this invention leads to the selective production of $\beta$-substituted naphthalenes such as 2-methylnaphthalene, and $\beta,\beta'$ disubstituted products such as 2,6-dimethylnaphthalene and 2,7-dimethylnaphthalene.

The process of this invention is further described and illustrated in the following examples 1 to 11. Examples A, B and C are comparative examples of a direct methylation process not according to this invention.

All the experiments hereinafter described except examples 5 and 11 were carried out in a laboratory microreactor.

The catalyst used in examples A, B and 1 to 4 was a sample of zeolite ZSM-5 having a silica to alumina mole ratio of 100:1 and a crystallite size of 1$\mu$.

EXAMPLE A

This example illustrates the direct methylation of a 3:1 solar mixture of 2-methylnaphthalene and 1-methylnaphthalene which was passed through the microreactor at a rate of 50 mmol/h with 50 mmol/h methanol and 500ml/h of nitrogen. The reactor contained 10 mls of zeolite ZSM-5 as a catalyst and the reaction temperature was 450° C.

Methanol conversion was 100%, mainly to olefins, paraffins and non-naphthalene aromatics. The proportions of naphthalene products obtained after 15 minutes reaction were as follows (in mol %).

| | |
|---|---|
| Naphthalene | 1.5 |
| 2-methylnaphthalene | 67.7 |
| 1-methylnaphthalene | 26.8 |
| Dimethylnaphthalenes | 2.7 |
| Ethylmethylnaphthalenes | 1.0 |
| Propylmethylnahthalenes | 0.3 |

EXAMPLE B

Example A was repeated except that the reaction temperature was 350° C. As in Example A, methanol conversion was 100%, mainly to olefins, paraffins and aromatics. The proportions of the naphthalene products obtained after 30 minutes reaction were as follows (in mol %):

| | |
|---|---|
| Naphthalene | 0.4 |
| 2-methylnaphthalene | 71.1 |
| 1-methylnaphthalene | 25.3 |
| Dimethylnaphthalenes | 1.2 |
| Ethylmethylnaphthalenes | 1.6 |
| Propylmethylnaphthalenes | 0.4 |

These results show that under the conditions of this experiment the formation of ethylmethyl naphthalene exceeds that of $\beta,\beta'$-dimethylnaphthalene.

EXAMPLE C

This comparative example illustrates the methylation of naphthalene using xylenes as the methylating agent and an amorphous silica-alumina catalyst. The experiment was carried out at 400° C in a flow system similar to that used for the examples of the process of the invention, as hereinafter described. Over the silica-alumina catalyst (10g) was passed a stream consisting of nitrogen (650 ml/hr), naphthalene (12.5 mmol/hr) and para-xylene (50 mmol/hr. After 3 hours the proportions of naphthalene compounds (mol %) in the product stream were:

| | |
|---|---|
| Naphthalene | 91.4 |
| 2-methyl naphthalene | 5.37 |
| 1-methyl naphthalene | 2.41 |

As well as xylenes at equilibrium, the single ring aromatic compounds present in the product stream were mainly toluene and benzene.

The results of this experiment show that 1-methyl naphthalene and 2-methylnaphthalene are produced at a ratio near to equilibrium, selectivity to the β-product being absent.

EXAMPLE 1

This example illustrates the methylation of 2-methylnaphthalene with xylenes. The reaction was carried out in the microreactor at 400°C over 10 ml of zeolite ZSM-5 catalyst and at flow rates of 12.5 mmol/h for the methylnaphthalene, 50 mmol/h for o-xylene and 500 ml/hr for diluent nitrogen.

In the product the single ring products were mainly a close-to-equilibrium mixture of o-, m- and p-xylene together with toluene and traces of benzene and 1,2,4-trimethylbenzene. The proportions of naphthalene products obtained after 15 minutes reaction were (in mol %).

| | |
|---|---|
| **Naphthalene** | **3.4** |
| **2-methylnaphthalene** | **84.7** |
| **1-methylnaphthalene** | **6.2** |
| **2,6 dimethylnaphthalene-rich** | **5.7** |
| **dimethylnaphthalenes** | |

EXAMPLE 2

This example illustrates the methylation of naphthalene with xylenes. The reaction conditions and flow rates were the same as in example 1 with naphthalene replacing 2-methylnaphthalene.

The ortho-xylene was converted mainly to a close-to-equilibrium mixture of o-, m- and p-xylene together with some toluene and traces of benzene and 1, 2, 4-trimethylbenzene. The proportions of naphthalene products obtained after 15 minutes reaction time were (in mol %):

| | |
|---|---|
| Naphthalene | 81.8 |
| 2-methylnaphthalene | 12.5 |
| 1-methylnaphthalene | 2.1 |
| 2,6 dimethylnaphthalene-rich | 3.5 |
| dimethylnaphthalenes | |

EXAMPLE 3

This example demonstrates selectivity in the methylation of biphenyl using the same zeolite ZSM-5 catalyst as that employed in Examples 1 and 2. The selectivity is for 4-methylbiphenyl and 4,4'-dimethylbiphenyl. Ortho-xylene was fed to provide the xylenes methylating agent. The reaction was carried out in the micro-reactor at 400°C over 10 mls of ZSM-5 catalyst and at flow rates of 12.5 mmol/h for biphenyl, 50 mmol/h for o-xylene and 500 ml/h for diluent nitrogen.

The ortho-xylene was converted mainly to a close-to-equilibrium mixture of o-, m- and p-xylene together with some toluene and traces of benzene and 1,2,4-trimethyl benzene. The proportions (mole %) of biphenyl products obtained were:

| | |
|---|---|
| **Biphenyl** | **89.4** |
| **4-methylbiphenyl** | **5.7** |

Other methylbiphenyls     2.6

4,4'-dimethylbiphenyls     1.1.

Other dimethylbiphenyls     1.2

Ratio of 4-methylbiphenyl/

other methylbiphenyls     0.69

Ratio of 4,4'-dimethylbiphenyl/

other dimethylbiphenyls     0.48

EXAMPLE 4

This example illustrates naphthalene methylation feeding p-xylene to provide the methylation agent. On this occasion a different sample of zeolite ZSM-5 catalyst was used. A larger catalyst charge and lower liquid feed rates were used thereby enabling a higher conversion to be achieved. The example also demonstrates that the catalyst has an acceptable life-time.

The reaction was carried out in the microreactor at 400°C over 30 ml of zeolite ZSM-5 catalyst and at flow rates of 6.25 mmol/h for naphthalene, 25 mmol/h for p-xylene and 500 ml/h for diluent nitrogen.

Product samples were taken at intervals over a period of 3 days and in all samples the para-xylene was converted mainly to a close-to-equilibrium mixture of o-, m- and p-xylene together with some toluene and traces of benzene and 1,2,4-trimethyl benzene. The proportions of naphthalene products obtained are shown in the following Table:

TABLE 1

| Component (mol %) | Sample time (hours) | | |
|---|---|---|---|
| | 22 | 45 | 72 |
| Naphthalene | 68.9 | 75.4 | 79.6 |
| 2-methylnaphthalene | 20.0 | 16.9 | 14.0 |
| 1-methylnaphthalene | 4.4 | 2.2 | 1.6 |
| 2,6 -rich dimethyl-naphthalenes | 6.6 | 5.5 | 4.8 |
| trimethylnaphthalenes | 0.1 | – | – |
| Ratio of 2-methylnaphthalene/ 2-methylnaphthalene + 1-methylnaphthalene | 0.82 | 0.88 | 0.90 |

In this example the temperature was held constant and the rate of conversion was allowed to slowly fall with time. If desired, the conversion may be held at a constant rate by adjusting the temperature, as required, during the course of the reaction.

These examples show that $\beta$-selectivity can be achieved in naphthalene alkylation using a xylene as the alkylating agent and H-ZSM-5 as the zeolite catalyst. As hereinbefore mentioned it is somewhat unexpected that H-ZSM-5, a zeolite that can shape-selectively produce para-xylene in toluene disproportionation and methylation, can not only methylate naphthalene with methanol but also methylate naphthalene with xylenes. Moreover the methylation of naphthalene or methylnaphthalene with xylene using this catalyst give a clean reaction with co-production of toluene and benzene, both useful chemicals. The only undesirable by-product is a small amount of trimethylbenzene.

## EXAMPLE 5

A further embodiment of the invention comprises the methylation of biphenyl, as illustrated in the accompanying flow diagram. Biphenyl feedstock from a supply (not shown) enters the reactor 1 along line 2 while mixed xylenes from a supply which is not shown but which for example may be the separation stage of a BTX aromatics process enters along line 3. The reactor 1 contains a fixed bed of zeolite catalyst and is operated at a temperature in the range 250 to 300°C and at a pressure up to 20 atmospheres. The liquid residence time in the reactor is typically in the range of a few minutes to a few hours while a total molar feed rate of bicyclic hydrocarbons relative to that of xylenes is in the range 0.1:1 to 2:1, especially about 0.5:1, is appropriate. The products of the reaction together with unreacted feedstocks are removed from the reactor and passed along line 4 to a distillation unit 5.

A stream rich in the desired product, 4,4'dimethylbiphenyl (bp 295°C) is removed as bottoms product from the distillation unit while the undesired products comprising other alkylated biphenyls and unreacted xylenes and biphenyl are removed overhead and fed along line 6 to a second distillation unit 7. There benzene and toluene are removed overhead while unreacted biphenyl and the undesired alkylated isomers are recycled along line 8 to reactor 1.

The catalyst used in examples 6 to 9 was a sample of zeolite ZSM-5 doped with compounds of magnesium and phosphorus. It was prepared by impregnating the zeolite with aqueous diammonium hydrogen phosphate solution followed by drying at 120°C and then calcining at 500°C. After cooling, the sample was impregnated with aqueous magnesium acetate solution followed by drying at 120°C and calcination at 500°C. The catalyst was used in the form of 1/8-inch (32 mm) extrudates, bound with alumina and it contained 3.5 wt% phosphorus and 2.4 wt% magnesium.

## EXAMPLE 6

This example illustrates the use of the phosphorus/magnesium-containing ZSM-5 catalyst for the methylation of naphthalene using xylenes as the methylating agent. Nitrogen (650 ml/hr), naphthalene (12.7 mmol/hr) and para-xylene (50.7 mmol/hr) were flowed through a bed of the catalyst (25 g) at 400°C. After two hours the proportions (in mol %) of naphthalene compounds in the product stream were:

| | |
|---|---|
| Naphthalene | 85.5 |
| 2-methylnaphthalene | 10.5 |
| 1-methylnaphthalene | 0.46 |
| 2,6-dimethylnaphthalene-rich dimethylnaphthalenes | 2.7 |

The single ring aromatic compounds in the product stream consisted largely of an equilibrium mixture of xylenes together with toluene and some benzene.

## EXAMPLE 7

This example illustrates the selective methylation of 2-methylnaphthalene using xylenes as the methylating agent. The reaction conditions and flow rates were the same as in example 6 except that 2-methylnaphthalene replaced the naphthalene feed. After two hours the proportions (in mol %) of naphthalene compounds in the product stream were:

| | |
|---|---|
| Naphthalene | 1.59 |
| 2-methylnaphthalene | 92.4 |
| 1-methylnaphthalene | 2.36 |
| 2,6-dimethylnaphthalene-rich dimethylnaphthalenes | 3.56 |

The composition of the single-ring aromatic compounds in the product stream was substantially the same as in example 6.

EXAMPLE 8

This example illustrates the selective methylation of biphenyl using xylenes as the methylating agent. Nitrogen (650 ml/hr), para-xylene (50mmol/hr) and biphenyl (12.5 mmol/hr) were flowed through a bed (75 g) of the same catalyst as used in examples 6 and 7. After three hours the proportions (in mol %) of biphenyl compounds in the product stream were:

| | |
|---|---|
| Biphenyl | 82.1 |
| 4-methylbiphenyl | 12.2 |
| 3-methylbiphenyl | 2.78 |
| 2-methylbiphenyl | 0.25 |
| 4.4'-dimethylbiphenyl | 1.24 |

The composition of the single ring aromatic compounds in the product stream was substantially the same as in example 6.

EXAMPLE 9

This example illustrates the methylation of naphthalene using xylenes as the methylating agent in a very similar way to example 6. The same type of zeolitic catalyst was used and the reaction temperature and ratio of reactants were unchanged. However a larger amount of catalyst and lower flow rates were used so that the space velocity was only about 25% of that used in example 6. After 6 hours the proportions (in mol %) of naphthalene compounds in the product stream were:

| | |
|---|---|
| Naphthalene | 72.9 |
| 2-methylnaphthalene | 21.1 |
| 1-methylnaphthalene | 1.32 |
| 2,6-dimethylnaphthalene-rich dimethylnaphthalenes | 3.62 |

A further reduction in the space velocity led to the following proportions of naphthalene compounds in the product stream:

| Naphthalene | 65.2 |
|---|---|
| 2-methylnaphthalene | 24.9 |
| 1-methylnaphthalene | 3.66 |
| 2,6-dimethylnaphthalene-rich dimethylnaphthalenes | 5.17 |

Examples 6 to 9 illustrate the $\beta$-selectivity of the process of the invention and example 9 in particular shows that high $\beta$-selectivity is retained even at high conversion of the compound undergoing alkylation.

EXAMPLE 10

This example illustrates the use of zeolite ZSM-11 as a catalyst for the selective methylation of naphthalene using xylenes as the methylating agent.

Nitrogen (650 ml/hr), naphthalene (12.5 mmol/hr) and para-xylene (49.8 mmol/hr) were flowed through a bed of pelleted ZSM-11 catalyst (7.8 g) at 400°C. After 1 hour the proportions (in mol %) of naphthalene compounds in the product stream were:

| Naphthalene | 85.6 |
|---|---|
| 2-methylnaphthalene | 8.55 |
| 1-methylnaphthalene | 1.98 |
| 2,6 dimethylnaphthalene-rich dimethylnaphthalenes | 3.51 |

EXAMPLE 11

This example illustrates a liquid-phase embodiment of the process of the invention. To an autoclave were charged ZSM-5 zeolite catalyst (10 g), naphthalene (5 g) and ortho-xylene (95 g). Hydrogen was introduced at 100 atmospheres and the reaction mixture heated to 325°C for 4 hours. After cooling and depressurising, the only naphthalene derivatives which were detected were 1- and 2-methyl-naphthalene in ratio 1:20 respectively, corresponding to 2.0% of the naphthalene originally charged.

The products of the process of this invention are believed to be of value as chemical intermediates. In particular it is believed that dialkylated biphenyls and naphthalenes will be valuable raw materials for the production of the corresponding dicarboxylic, dihydroxy and monocarboxylic-monohydroxy derivatives, all having potential as the monomers of high value polymers.

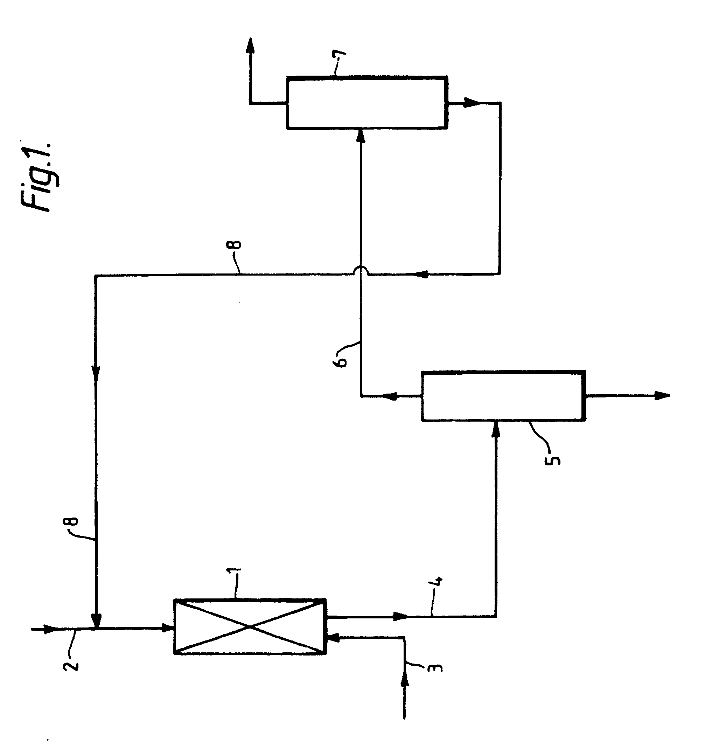

**Claims**

1. A process for the selective production of $\beta$-substituted or $\beta,\beta'$-disubstituted alkylated derivatives of naphthalene or a naphthalene derivative or 4-substituted or 4,4'-disubstituted alkylated derivatives of biphenyl or a biphenyl derivative which comprises contacting naphthalene or a naphthalene derivative or biphenyl or a biphenyl derivative with an alkylated benzene in the presence of a catalyst comprising a crystalline medium-pore zeolite or zeolitic material.

2. A process as claimed in claim 1 wherein the alkylated benzene comprises a xylene or mixture of xylenes.

3. A process as claimed in any one of the preceding claims wherein the zeolite comprises a zeolite of the ZSM-5 type or ZSM-11

4. A process as claimed in claim 3 wherein the zeolite comprises a zeolite containing a proportion of magnesium and/or phosphorus compounds.

5. A process for producing β-substituted or β,β'-disubstituted alkylated derivatives of naphthalene or a naphthalene derivative or 4-substituted or 4,4'-disubstituted alkylated derivatives of biphenyl or a biphenyl derivative which comprises;

(a) feeding to a reactor a first feedstock which comprises naphthalene or a naphthalene derivative or biphenyl or a biphenyl derivative and a mixed xylenes feedstock;

(b) contacting, as claimed in claim 1, the feedstocks together in the presence of a crystalline medium-pore zeolite or zeolitic material;

(c) passing the product of stage (b) to first distillation;

(d) recovering desired products as bottoms;

(e) passing undesired products overhead to second distillation;

(f) recovering as bottoms unreacted feedstock material or alkylated isomers of desired products;

(g) recycling such bottoms to stage (b).

**Revendications**

1. Procédé pour la production sélective de dérivés alkylés β-substitués ou β, β'-disubstitués de naphtalène ou d'un dérivé de naphtalène ou de dérivés alkylés 4-substitués ou 4,4'-disubstitués de biphényle ou d'un dérivé de biphényle, caractérisé en ce qu'il comprend la mise en contact du naphtalène ou d'un dérivé du naphtalène ou du biphényle ou d'un dérivé du biphényle avec un benzène alkylé en présence d'un catalyseur comprenant une matière zéolitique ou une zéolite cristalline à pore moyen.

2. Procédé suivant la revendication 1, caractérisé en ce que le benzène alkylé comprend un xylène ou un mélange de xylènes.

3. Procédé suivant l'une quelconque des revendications précédentes, caractérisé en ce que la zéolite comprend une zéolite du type ZSM-5 ou ZSM-11.

4. Procédé suivant la revendication 3, caractérisé en ce que la zéolite comprend une zéolite contenant une proportion de composés du magnésium et/ou du phosphore.

5. Procédé pour produire des dérivés alkylés β-substitués ou β,β'-disubstitués du naphtalène ou d'un dérivé du naphtalène ou des dérivés alkylés 4-substitués ou 4,4'-disubstitués du biphényle ou d'un dérivé du biphényle caractérisé en ce qu'il comprend :

(a) l'introduction dans un réacteur d'une première charge d'alimentation qui comprend du naphtalène ou un dérivé du naphtalène ou du biphényle ou un dérivé du biphényle et une charge d'alimentation de xylènes mélangés ;

(b) la mise en contact, suivant la revendication 1, des charges d'alimentation en présence d'une matière zéolitique ou d'une zéolite cristalline à pore moyen ;

(c) l'envoi du produit de l'étape (b) vers une première distillation ;

(d) la récupération des produits désirés en tant que queues ;

(e) l'envoi des produits indésirés en tête vers une seconde distillation ;

(f) la récupération en queue de la matière d'alimentation n'ayant pas réagi ou des isomères alkylés des produits désirés ;

(g) le recyclage de ces queues vers l'étape (b).

**Patentansprüche**

1. Verfahren zur selektiven Herstellung von β-substituierten oder β,β'-disubstituierten alkylierten Derivaten von Naphthalin oder einem Naphthalinderivat oder von 4-substituierten oder 4, 4'-disubstituierten alkylierten Derivaten von Biphenyl oder einem Biphenylderivat, bei dem Naphthalin oder ein Naphthalinderivat oder Biphenyl oder ein Biphenylderivat in Gegenwart eines Katalysators, der einen kristallinen Zeolithen mit mittlerem Porendurchmesser oder eine kristalline zeolithische Substanz mit mittlerem Porendurchmesser enthält, mit einem alkylierten Benzol in Berührung gebracht wird.

2. Verfahren nach Anspruch 1, bei dem das alkylierte Benzol aus einem Xylol oder aus einer Mischung von Xylolen besteht.

3. Verfahren nach einem der vorhergehenden Ansprüche, bei dem der Zeolith aus einem Zeolithen des ZSM-5-Typs oder ZSM-11 besteht.

4. Verfahren nach Anspruch 3, bei dem der Zeolith aus einem Zeolithen besteht, der einen Anteil von Magnesium- und/oder Phosphorverbindungen enthält.

5. Verfahren zur Herstellung von β-substituierten oder β,β'-disubstituierten alkylierten Derivaten von Naphthalin oder einem Naphthalinderivat oder von 4-substituierten oder 4,4'-disubstituierten alkylierten Derivaten von Biphenyl oder einem Biphenylderivat, bei dem:

(a) einem Reaktionsbehälter ein erstes Ausgangsmaterial, das aus Naphthalin oder einem Naphthalinderivat oder aus Biphenyl oder einem Biphenylderivat besteht, und ein Ausgangsmaterial aus gemischten Xylolen zugeführt werden,

(b) die Ausgangsmaterialien miteinander wie in Anspruch 1 beansprucht in Gegenwart eines kristallinen Zeolithen mit mittlerem Porendurchmesser oder einer kristallinen zeolithischen Substanz mit mittlerem Porendurchmesser in Berührung gebracht werden,

(c) das Produkt von Stufe (b) einer ersten Destillation zugeführt wird,

(d) gewünschte Produkte als Sumpfprodukte gewonnen werden,

(e) unerwünschte Produkte über Kopf einer zweiten Destillation zugeführt werden,

(f) unumgesetztes Ausgangsmaterial oder alkylierte Isomere gewünschter Produkte als Sumpfprodukte gewonnen werden,

(g) solche Sumpfprodukte zu Stufe (b) zurückgeführt werden.

Fig.1.